# EUROPEAN PATENT SPECIFICATION

(11) **EP 2 157 990 B1**
(45) Date of publication and mention of the grant of the patent: **30.07.2014**
(21) Application number: 08766981.8
(22) Date of filing: 29.05.2008
(51) Int. Cl.: A61M 15/00

(54) **INHALER FOR POWDER DRUG ADMINISTRATION**
INHALATOR ZUR VERABREICHUNG EINES PULVERFÖRMIGEN MEDIKAMENTS
INHALATEUR POUR UNE ADMINISTRATION DE MÉDICAMENT PULVÉRULENT

(30) Priority: 18.06.2007 PL 38268407
(43) Date of publication of application: 03.03.2010
(73) Proprietor: Przedsiebiorstwo Produkcji, 51-131 Wroclaw (PL)
(72) Inventor: GRADON, Leon, PL-02-796 Warszaw (PL); SOSNOWSKI, Tomasz, PL-01-917 Warszawa (PL); RUMINSKI, Witalis, PL-03-982 Warszawa (PL); SZWAST, Maciej, PL-02-784 Warszawa (PL); PIROZYNSKI, Michat, PL-00-358 Warszawa (PL); HAN, Stanislaw, PL-51-604 Wroclaw (PL)
(74) Representative: Krekora, Magdalena
(86) International application number: PCT/PL2008/000039
(87) International publication number: WO 2008/156381

(56) References cited:
- EP-A- 0 271 029
- WO-A-01/00262
- WO-A-03/075988
- WO-A-2004/011071
- GB-A- 2 367 756
- US-A- 3 921 637
- US-A- 4 069 819

## Description

The present invention relates to an inhaler for powder drug administration, applicable both for administration of drugs in the respiratory system therapy and for systemic administration of drugs.

A drug finding its way to a respiratory system is in aerosol form, i.e. in form of a lasting dispersion of liquid drops or solids in the inhaled air. A liquid drug is transformed into an aerosol by atomizing the liquid using ultrasounds or by dispersing it by compressed air while passing through a nozzle. A solid drug to be first reduced to powder and then its dispersion in a volatile medium first formed under elevated pressure, is expanded on a nozzle, thus transforming into an aerosol. Volatile matter evaporates, and particulates form an aerosol which is inhaled by a patient. Lack of synchronization between breathing-in and the moment of the aerosol release from the pressurised container as well as high rate of aerosol flow out of the nozzle result in that the aerosol flow will not be effectively directed by breath towards the trachea, thus striking hard against a rear wall of the throat. So undesired medicine deposition will follow in this region of the respiratory system, and only a little part of the medicine will get to a bronchial tree.

The therapeutic effect of inhalation depends on size of aerosol particles, and thus on the original size of powder particles. Proper effects are obtained for the aerosol particle diameters under 5 µm. Present trends and engineering opportunities indicate improved effectiveness of the aerosol therapy when the particle diameters in the powder of which the aerosol is formed are on the order of hundreds of nanometers. For fine break-up of active substance, its particles are deposited over a surface of larger particles of pharmacologically neutral substance. During inhalation, if using an inhaler of a suitable design, particles of active substance become separated from carrier. Large diameter carrier particles a will be captured in head airways, while the smaller drug particles penetrate to further parts of the respiratory system. If the powder is formed of drug substance particles, they hold together in the powder structure by cohesion forces, which strongly unite molecules together. To carry away particles of such structure, quite a high energy of the flowing-through air is needed. This can be achieved by special design of an applicator for powder transformation into the aerosol form.

The powder either as pure active substance or in form of carrier-bonded particles of the same substance is fed to a powder inhaler from a container in form of a capsule or blister. A portion of drug contained in the container is determined by size of applied dose. Effectiveness of emptying the container of the portion of powder contained therein is an important feature in the powder inhaler design. Effectiveness of emptying the container and transforming the powder into aerosol form certifies of the inhaler quality.

A powder inhaler as known from Polish patent application No. 370285 is intended for administration of drugs in patients with substantially impaired respiratory system. The inhaller has an aerodynamic chamber and air inlet passages shaped in such a way that the air flowing therethrough forms at least three groups of streams, where at least one of them flows into the chamber from the opposite end to the outlet of the chamber, and at least two of them flow in symmetrically from both sides, at acute angle to the axial centre line of the aerodynamic chamber. Centre lines of air streams flowing into the aerodynamic chamber intersect before reaching the place where the drug powder is carried away, producing thereby strongly turbulent flow even at low rates, owing to which the inhalation proceeds correctly.

Another powder inhaler - a capsule opening and emptying system - is known from the Polish patent application No. 370286. The inhaler for inhalation of powdered drugs, provided with a system for emtying drug capsules has an aerodynamic chamber wherein the powder for inhalation is placed. The system for emtying drug capsules is composed of a motionless part - containing a tool for emptying the capsules and, possibly, a safety partition; and of a moving part - consisting of a capsule receptacle and a driving mechanism.

There is a number of design solutions for powder drug inhalers. The patent specifications US 3,991,761, EP 0005585 and PCT/EP2005/005182 disclose powder inhalers wherein a medication containing capsule is placed in a chamber extended in a form of a mouthpiece, and the inspired air flows there through. The drug containing capsule is opened by puncture its rounded ends with needles. As a result of the capsule rotation caused by the flowing through air, the powder is released from the capsule through the made holes. Agglomerates formed of drug particles and the carrier undergo breaking on a perforated partition between the capsule chamber and the air passage formed as a mouthpiece. Disadvantage of such solutions consists in low effectiveness of capsule emptying and in its uncertainty, due to which the reproducibility of medication doses is low. Besides, a great deal of air flow energy is consumed for movement of a capsule. Thus the air flow resistance is high, and in consequence such solutions can not be used in patients having badly damaged respiratory function. A variant of this type of inhaler is disclosed in WO63/075988.

Another powder inhaler was disclosed in the patent specification GB 2165159, where a definite portion of drug is dosed from a container into the inhalation chamber by way of a specially shaped knob. There is an air inlet to the inhalation chamber and a passage to deliver aerosol to the respiratory system. Drug aerolization and breaking follows in the effect of the flow turbulence generation on a reducer. Such effect can be obtained only at a very intensive flow rate of the inspired air. Therefore this solution cannot be used in patients having weak inspiration, e.g. children or elderly people.

A powder inhaller according to the patent specification PCT WO03/103563 is designed with a passage, and the air enters the passage through many openings spaced along the passage. The air flow rate through the passage can be adjusted by covering respective openings with fingers while taking a breath. This way however the uncertainty of the air flow is high, which results in lack of reproducibility of the medication doses inspired during consecutive inhalations.

A powder inhaler, wherein powder aerolization takes place as the compressed air stream, inflowing from an attached tank, flows through a drug-containing capsule, was disclosed in the WO2005/084735 patent specification. In this solution, as well as in case of pressure inhalers that dose suspended drugs, it is necessary to synchronize the moments of taking a breath and actuating the inhaler. In order to improve breaking of the powder agglomerates, the latter inhaler has been provided with a narrow-passage nozzle. However, such solution requires that the air delivered from an attached air tank to be under much higher positive gauge pressure, which brings about additional constructional problems.

The essence of the inhaler according to the invention is specified in claim 1. The aerosolization chamber at the top is joined to a capsule emptying chamber, and this chamber in turn is closed from the top with a lid serving also as a capsule holder, whereas the air inlet, through which the compressed air stream enters the aerosolization chamber tangently to its preferably concave bottom, is attached to a source of air for aerosolization, and a partition is provided in the aerosol outlet. There is a cavity in the bottom surface of the capsule holder, matching the drug capsule shape, preferably located centrally in the capsule holder.

The air inlet consists preferably of at least one slot, located at the bottom of the cylindrical aerosolization chamber and along a part of its circumference formed by opening angle not exceeding 120°.

Preferably the air inlet consists of a set of openings located at the bottom of the cylindrical aerosolization chamber, within a sector corresponding to an opening angle not less than 30° and not exceeding 120°.

Preferably the compressed air enters the aerosolization chamber in pulses from the source of air for aerosolization, through a shut-off valve mounted between the air inlet and the source of air for aerosolization. Volume of the air delivered into the aerosolization chamber, after expansion to standard conditions will exceed the volume of the aerosolization chamber.

There is a shearing tool mounted in a side wall of the body of the capsule emtying chamber, and outside air inlet openings go through the side walls of the body, in order to ensure the outside air flow into the inhaler.

A guide provided with a return spring is seated preferably in a side wall of the body, and the shearing tool is mounted in the said guide and connected to a button located outside the inhaler. Further, a stabilizer of the capsule position is mounted in the capsule emptying chamber, where the said stabilizer locks the capsule in place when the capsule is pressed with the a shearing tool.

Also preferable is that the said guide provided with a return spring is seated in a side wall of the body, and the shearing tool is mounted inside the said guide and connected to the outside button, whereas a container releasing a strip of blisters, where each blister contains a dose of the administered medication, is located above the capsule emptying chamber.

Preferably the source of air for aerosolization consists of a container, with a piston placed therein, and the said piston is connected to a power spring and a system of tension and release of the said spring, and the said container is attached to the aerosolization chamber through a joining passage.

It is also preferable that the source of air for aerosolization consists of a container with a piston placed therein, where the said piston is driven by a power spring and actuated by a spring tension and release system, and the said piston is set on a shaft aligned axially in the said cylindrical container. The said container is attached to the aerosolization chamber through a joining passage.

Preferably the aerosolization chamber, the capsule emptying chamber and the capsule holder are joined to each other tight.

Preferably, a cross-section of the aerosolization chamber at a level of the aerosol outlet is larger than the aerosolization chamber cross-section at the air inlet level.

The advantage of the powder drug inhaler according to the invention is its simple design, facilitating a correct course of inhalation and a good deposition of medication in the bronchial tree and pulmonary alveoli, particularly in patients with damaged respiratory function. The novel inhaler provides for an effective and reproducible emptying of a powder medication container, and for powder transformation into an aerosol form through delivery of additional air stream from the source of air for use in the aerosolization process, where the said air source is attached to this inhaler, and further this novel inhaler provides for inspiration of such formed aerosol into patient's lungs.

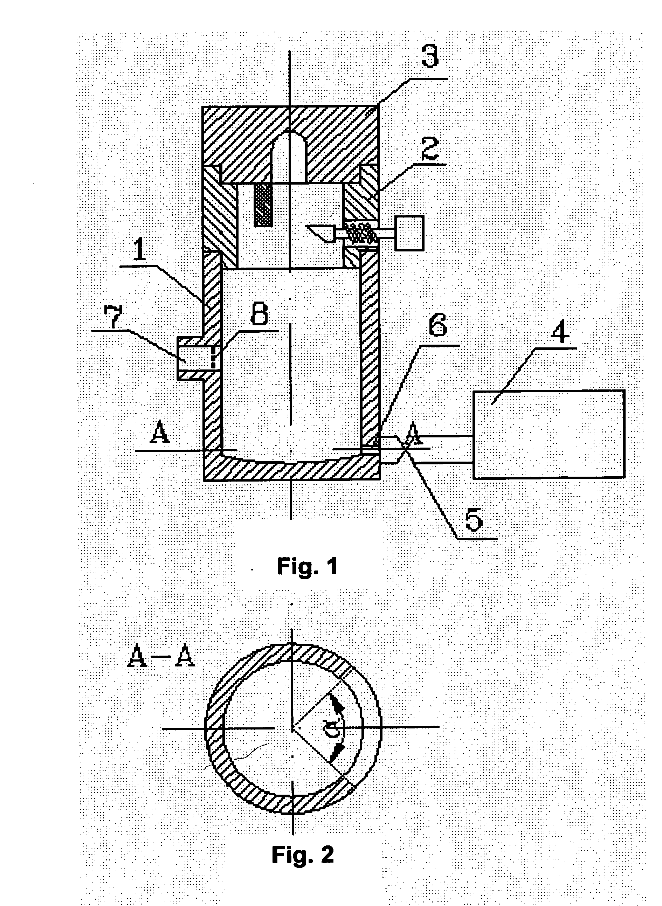

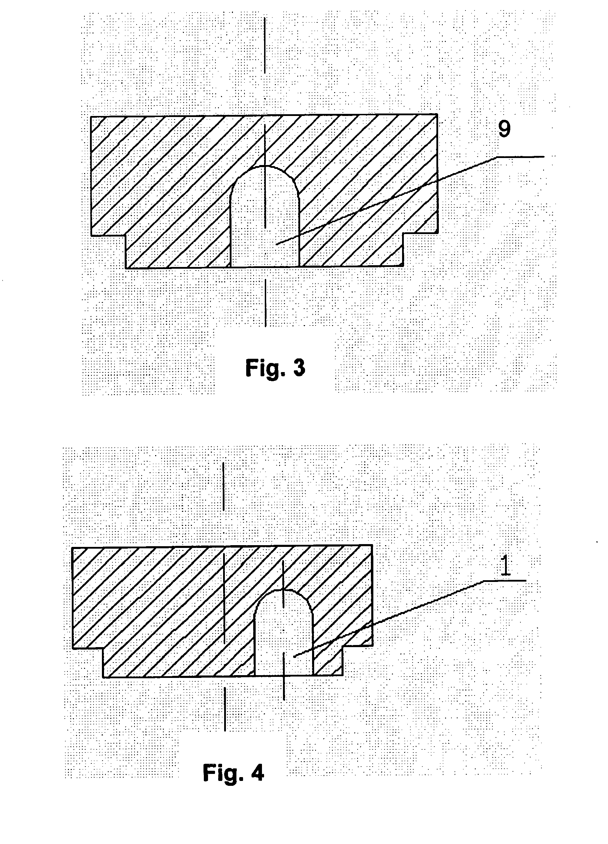

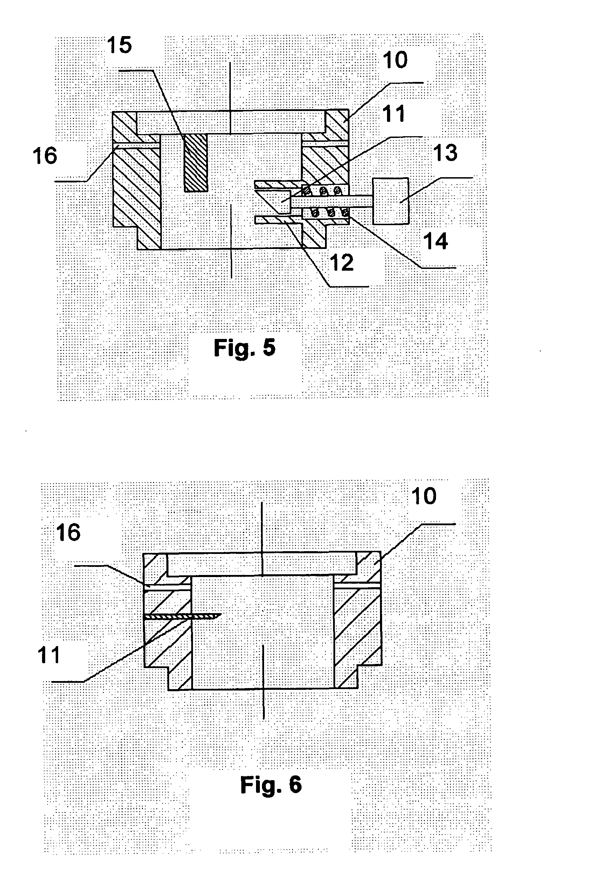

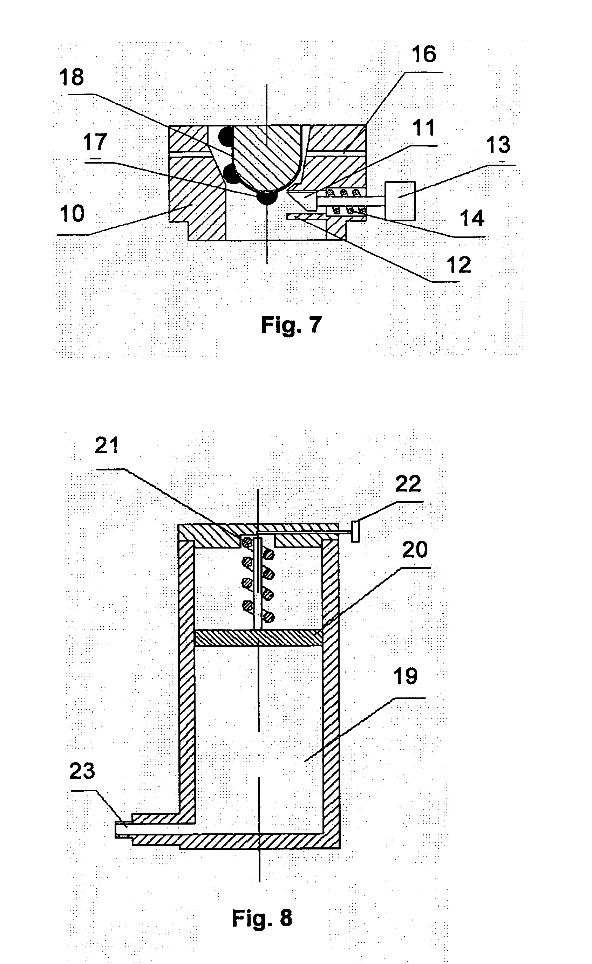

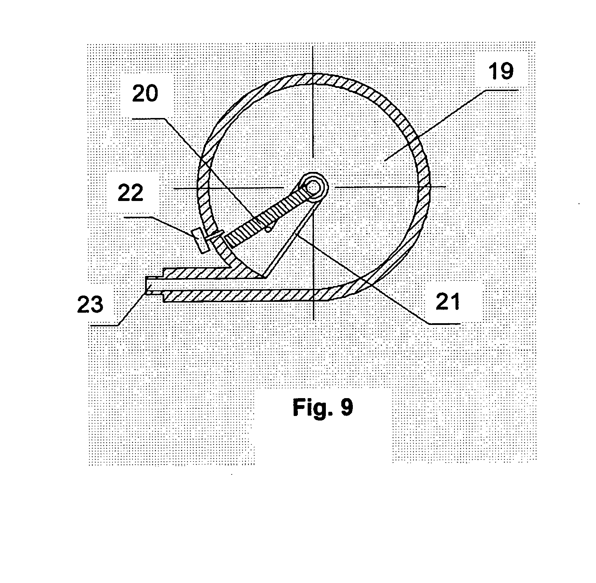

The object of the invention as an exemplary embodiment has been shown in the drawings: Fig.1 - axial sectional view of the inhaler provided with a cylindrical aerosolization chamber; Fig.2 - inhaler cross-section in the A-A plane of the air inlet; Fig.3 - capsule holder with a centrally located cavity; Fig.4 - capsule holder with an asymmetrically located cavity; Fig.5 - capsule emptying chamber, shearing tool seated in a side wall of the chamber; Fig.6 - capsule emptying chamber, shearing tool mounted into a guide; Fig.7 - capsule emptying chamber, shearing tool mounted into a guide, the chamber provided with a container to release a blister strip; Fig.8 - source of air for aerosolization, provided with a piston connected to a power spring and to a spring tension and release system; Fig. 9 - source of air for aerosolization, provided with a piston driven by a power spring and actuated by a spring tension and release system, where the piston is set on a shaft aligned axially in the cylindrical container.

### Example 1

The inhaler for administration of powder drugs has an aerosolization chamber 1 which is closed from the top with a lid, serving as a capsule holder 3. Compressed air from the source of air for aerosolization 4 is delivered to the aerosolization chamber 1 through a shut-off valve 5 mounted between the air inlet 6 and the source of air for aerosolization 4. Through the air inlet 6, made in a side wall of the aerosolization chamber 1, and entering the source of air for aerosolization 4, the compressed air stream is delivered tangently to the bottom of the aerosolization chamber 1. Volume of air delivered into the aerosolization chamber 1, after expansion to standard conditions will exceed the volume of the aerosolization chamber 1. The air is taken off the aerosolization chamber 1 through the aerosol outlet 7, which is made in a side wall of the aerosolization chamber 1 on the opposite side to the air inlet 6, and the said aerosol outlet 7 is provided with a partition 8. In this exemplary embodiment the air inlet 6 constitutes a slot located at the bottom of the cylindrical aerosolization chamber 1 and along a part of its circumference formed by an opening angle α which is equal to 120°. The aerosolization chamber 1 is attached tight at the top to a capsule emptying chamber 2, and the said capsule emptying chamber 2 has a body 10, in the side wall whereof mounted is a shearing tool 11. Outside air inlet openings 16 are made in side walls of the body 10 in order to ensure the outside air flow into the inhaler at the time when the aerosol formed in the aerosolization chamber 1 is being inspired. The capsule emptying chamber 2 is closed tight by the capsule holder 3, having on its bottom side a cavity 9, in shape of a capsule with the administered medication. The cavity 9 is situated asymmetrically, and a larger part of the capsule placed therein protrudes outwards the cavity 9; at the moment when the capsule holder 3 is rotated, the protruding part of the capsule is shorn off by a fixed shearing tool 11, that serves to empty the capsules. The bottom part of the capsule, together with contained medication, falls down into the aerosolization chamber 1, wherefrom the drug powder is aerolized.

### Example 2

The inhaler for administration of powder drugs, made as in the first example, except that a guide 12 provided with a return spring 14 is seated in a side wall of a body 10 of the capsule emptying chamber 2, whereas the shearing tool 11 is connected to a button 13 located on the outside, and is mounted in the said guide 12. Further a capsule position stabilizer 15 is mounted in the capsule emptying chamber 2, and it locks the capsule in place when the capsule is pressed by the said shearing tool 11. Further a container 19 attached to the aerosolization chamber 1 through a joining passage 23, serves as a source of air for aerosolization 4. There is a piston 20 in the container 19, and the said piston 20 is connected to a power spring 21 and to a spring tension and release system 22. The compressed air is let into the aerosolization chamber 1 in pulses from the source of air for aerosolization 4, through a shut-off valve 5 mounted between the air inlet 6 and the source of air for aerosolization 4, where the air inlet 6 consists of a set of openings located at the bottom of the cylindrical aerosolization chamber 1, within a sector corresponding to an opening angle α equal to 30°. The aerosolization chamber 1 joins through the aerosol outlet 7 to a mouthpiece or a tip delivering the aerosol into a nasal passage.

### Example 3

The inhaler for administration of powder drugs, made as in the first or second example, except that the cavity 9 is located centrally in the capsule holder 3, and that the guide 12 provided with a return spring 14 is seated in a side wall of a body 10 of the capsule emptying chamber 2, whereas the shearing tool 11 connected to a button 13 located on the outside, is mounted in the said guide 12. There is a container 19 above the capsule emptying chamber 2, releasing a strip 18 of blisters 17, where each blister 17 contains a dose of the administered medication. While the source of air for aerosolization 4 constitutes a container 19 with a piston 20 inside, where the said piston 20 is driven by a power spring 21 and actuated by a spring tension and release system 22. The piston 20 is set on a shaft aligned axially in a cylindrical container 19 and moves angularly round this axis, while the container 19 is attached to the aerosolization chamber 1 through a joining passage 23. Further a bottom of the aerosolization chamber 1 is concave in shape, therefore a dose of medication powder, when poured out of a capsule, or in case of incidental change of the inhaler position during preparation for inhalation, will not shift freely over the bottom of the aerosolization chamber 1.

This inhaler model is fit for dosing medication packed in blisters. A dose of medication in a blister 17 placed on a strip 2 enters an area of the body 10 above the aerosolization chamber 1. Next, on cutting down the bottom part of the blister 17 with a shearing tool 11, which is moving in a guide 12, the powder pours out. As the strip 18 is rigid, the blister 17 holds in position while being cut down with a shearing tool 11.

The aerosolization chamber 1 is attached to a capsule emptying chamber 2 provided with a capsule opening device comprising a shearing tool 11, where the said shearing tool 11 opens a capsule by cutting down its bottom part. Once that part of the capsule has been cut down, the powder medication pours out over a concave surface of the bottom of the aerosolization chamber 1. Air from the attached source of air for aerosolization 4 passes through a shut-off valve 5 and enters the aerosolization chamber 1 through a slotted air inlet 6 located at the bottom of the aerosolization chamber 1 tangently to its bottom. The flowing air, owing to its kinetic energy, carries away the powder particles. Strong vortex and blow of the air stream against a wall located opposite to the air inlet in the aerosolization chamber 1, stimulates turbulence of movement of the air carrying away powder particles. Kinetic energy of the turbulent air movement in the aerosolization chamber 1 causes additional breaking of the agglomerates of drug particles, which produces an aerosol that contains particles of size originally existing in the powder structure. In case of agglomerates formed of particles and the carrier, there follows an effective separation of active substance from the carrier. The aerosol produced in the aerosolization chamber 1 is inhaled into the system through a mouthpiece or a nasal tip. Energy of inspiration is necessary only to introduce the aerosol into the respiratory system. The air for aerosolization can be taken also from a compressed air container attached to the aerosolization chamber 1, through a shut-off valve 5, or can be introduced into the chamber by a dynamic method through a mechanical air injection into the aerosolization chamber 1.

### Example 4

The inhaler for administration of powder drugs, made as in the first or second or third example, except that the cross-section of the aerosolization chamber 1 at the level of the aerosol outlet 7 is larger than the cross-section of the aerosolization chamber 1 at the air inlet 6 level, and that the air inlet 6 consists of thirty slots located at the bottom of the cylindrical aerosolization chamber 1 and along a part of its circumference formed by opening angle α equal to 120°, whereas the breadth of each slot corresponds to the length of a part of the circumference of the aerosolization chamber 1, formed by opening angle α equal to 2°.

## Claims

1. An inhaler for administration of powder drugs provided with an aerosolization chamber wherein the powder for inhalation is placed, and the air inlet to the chamber and the air outlet from the chamber located on the opposite sidewalls of the aerozolization chamber, **characterised in that** the aerosolization chamber (1) is attached at the top to a capsule emptying chamber (2), which is covered from the top with a lid serving as a capsule holder (3), whereas the air inlet (6), configured to allow a compressed air stream to enter the aerosolization chamber (1) tangently to its bottom, makes a joining passage to the source of air for aerosolization (4), while a partition (8) is provided in the aerosol outlet (7).

2. An inhaler according to claim 1, **characterised in that** the air inlet (6) consists of at least one slot located at the bottom of the cylindrical aerosolization chamber (1) and along a part of its circumference formed within an opening angle (α) not exceeding 120°.

3. An inhaler according to claim 1, **characterised in that** the air inlet (6) consists of a set of openings located at the bottom of the cylindrical aerosolization chamber (1), within a sector corresponding to an opening angle (α) not less than 30° and not exceeding 120°.

4. An inhaler according to claim 1, **characterised in that** the bottom of the aerosolization chamber (1) is concave in shape.

5. An inhaler according to claim 1, **characterised in that** the compressed air enters the aerosolization chamber (1) in pulses from the source of air for aerosolization (4), through a shut-off valve (5) mounted between the air inlet (6) and the source of air for aerosolization.

6. An inhaler according to claim 1, **characterised in that** the volume of the air delivered into the aerosolization chamber (1), after expansion to standard conditions will exceed the volume of the aerosolization chamber (1).

7. An inhaler according to claim 1, **characterised in that** there is a cavity (9) in the bottom surface of the capsule holder (3), matching the shape of the capsule that contains the administered drug.

8. An inhaler according to claim 7, **characterised in that** the cavity (9) is located centrally in the capsule holder (3).

9. An inhaler according to claim 1, **characterised in that** the capsule emptying chamber (2) is provided with a body (10), and a shearing tool (11) is mounted in a side wall thereof, whereas outside air inlet openings (16) are made in side walls of the body (10) in order to ensure the outside air flow into the inhaler.

10. An inhaler according to claim 9, **characterised in that** a guide (12) provided with a return spring (14) is seated in a side wall of the body (10), and the shearing tool (11) connected to a button (13) placed on the outside is mounted in the guide (12), and further a stabilizer of the capsule position (15) is mounted in the capsule emptying chamber (2), and it locks the capsule in place when the capsule is pressed with the shearing tool (11).

11. An inhaler according to claim 9, **characterised in that** a guide (12) provided with a return spring (14) is seated in a side wall of the body (10), and the shearing tool (11) connected to a button (13) placed on the outside is mounted in the guide (12), whereas above the capsule emptying chamber (2) located is a container (19) that releases a strip (18) of blisters (17), where each blister (17) contains a dose of the administered medication.

12. An inhaler according to claim 1, **characterised in that** the source of air for aerosolization (4) constitutes a container (19), with a piston (20) placed therein and connected to a power spring (21) and to a system of spring tension and release (22), whereas the container (19) is attached to the aerosolization chamber (1) through a joining passage (23).

13. An inhaler according to claim 1, **characterised in that** the source of air for aerosolization (4) constitutes a container (19), with a piston (20) placed therein, where the piston (20) is driven by a power spring (21) and actuated by a spring tension and release system (22), and the piston (20) is set on a shaft aligned axially in the said cylindrical container (19), whereas the said container (19) is attached to the aerosolization chamber (1) through a joining passage (23).

14. An inhaler according to claim 1, **characterised in that** the aerosolization chamber (1), the capsule emptying chamber (2) and the capsule holder (3) are attached to each other tight.

15. An inhaler according to claim 1, **characterised in that** the cross-section of the aerosolization chamber (1) at the level of the aerosol outlet (7) is larger than the cross-section of the aerosolization chamber (1) at the air inlet (6) level.

## Patentansprüche

1. Inhalator zur Verabreichung von pulverförmigen Medikamenten, der mit einer Feinzerstäubungskammer versehen ist, in der das Pulver zur Inhalation platziert ist, wobei der Lufteinlass zur Kammer und der Luftauslass aus der Kammer an den gegenüberliegenden Seitenwänden der Feinzerstäubungskammer angeordnet sind, **dadurch gekennzeichnet, dass** die Feinzerstäubungskammer (1) an seiner Oberseite an einer Kapselleerungskammer (2) angebracht ist, die von oben mit einem Deckel abgedeckt ist, der als ein Kapselhalter (3) dient, wobei der Lufteinlass (6), der dazu konfiguriert ist, das Eintreten eines Druckluftstroms in die Feinzerstäubungskammer (1) tangential zu ihrem Boden zu gestatten, einen Verbindungsdurchgang zu der Luftquelle (4) für die Feinzerstäubung herstellt, während im Aerosolauslass (7) eine Trennwand (8) vorgesehen ist.

2. Inhalator nach Anspruch 1, **dadurch gekennzeichnet, dass** der Lufteinlass (6) aus mindestens einem Schlitz besteht, der am Boden der zylindrischen Feinzerstäubungskammer (1) angeordnet und entlang einem Teil seines Umfangs in einem Öffnungswinkel (α) von höchstens 120° gebildet ist.

3. Inhalator nach Anspruch 1, **dadurch gekennzeichnet, dass** der Lufteinlass (6) aus einem Satz Öffnungen besteht, die am Boden der zylindrischen Feinzerstäubungskammer (1) innerhalb eines Sektors angeordnet sind, der einem Öffnungswinkel (α) von mindestens 30° und höchstens 120° entspricht.

4. Inhalator nach Anspruch 1, **dadurch gekennzeichnet, dass** der Boden der Feinzerstäubungskammer (1) eine konkave Gestalt hat.

5. Inhalator nach Anspruch 1, **dadurch gekennzeichnet, dass** die Druckluft im Pulsbetrieb von der Luftquelle (4) für die Feinzerstäubung, durch ein zwischen dem Lufteinlass (6) und der Luftquelle für die Feinzerstäubung montiertes Absperrventil, (5) in die Feinzerstäubungskammer (1) eintritt.

6. Inhalator nach Anspruch 1, **dadurch gekennzeichnet, dass** das Volumen der in die Feinzerstäubungskammer (1) gelieferten Luft nach Expandierung auf Standardbedingungen das Volumen der Feinzerstäubungskammer (1) übersteigt.

7. Inhalator nach Anspruch 1, **dadurch gekennzeichnet, dass** in der Bodenfläche des Kapselhalters (3) ein Hohlraum (9) ist, dessen Gestalt der der Kapsel entspricht, die das verabreichte Medikament enthält.

8. Inhalator nach Anspruch 7, **dadurch gekennzeichnet, dass** der Hohlraum (9) mittig im Kapselhalter (3) angeordnet ist.

9. Inhalator nach Anspruch 1, **dadurch gekennzeichnet, dass** die Kapselleerungskammer (2) mit einem Körper (10) versehen ist und ein Abscherwerkzeug (11) in einer Seitenwand davon montiert ist, während Außenlufteinlassöffnungen (16) in Seitenwänden des Körpers (10) ausgeführt sind, um die Außenluftströmung in den Inhalator hinein zu gewährleisten.

10. Inhalator nach Anspruch 9, **dadurch gekennzeichnet, dass** eine mit einer Rückstellfeder (14) vorgesehene Führung (12) in einer Seitenwand des Körpers (10) sitzt und das Abscherwerkzeug (11), das mit einer außer dem Körper platzierten Taste (13) verbunden ist, mindestens teilweise in der Führung (12) montiert ist, und dass ausserdem ein Stabilisator der Kapselposition (15) in der Kapselleerungskammer (2) montiert ist und die Kapsel an Ort und Stelle verriegelt, wenn mit dem Abscherwerkzeug (11) auf die Kapsel gedrückt wird.

11. Inhalator nach Anspruch 9, **dadurch gekennzeichnet, dass** eine mit einer Rückstellfeder (14) vorgesehene Führung (12) in einer Seitenwand des Körpers (10) sitzt und das Abscherwerkzeug (11), das mit einer außer dem Körper platzierten Taste (13) verbunden ist, mindestens teilweise in der Führung (12) montiert ist, während über der Kapselleerungskammer (2) ein Behälter (19) angeordnet ist, der Schritt für Schritt einen Streifen (18) von Blistern (17) freigibt, wobei jeder Blister (17) eine Dosis des verabreichten Medikaments enthält.

12. Inhalator nach Anspruch 1, **dadurch gekennzeichnet, dass** die Luftquelle (4) für die Feinzerstäubung einen Behälter (19) darstellt, in dem ein Kolben (20) platziert ist, der mit einer Triebfeder (21) und mit einem Federspann-und-lösesystem (22) verbunden ist, während der Behälter (19) durch einen Verbindungsdurchgang (23) an der Feinzerstäubungskammer (1) angebracht ist.

13. Inhalator nach Anspruch 1, **dadurch gekennzeichnet, dass** die Luftquelle (4) für die Feinzerstäubung einen Behälter (19) darstellt, in dem ein Kolben (20) platziert ist, wobei der Kolben (20) von einer Triebfeder (21) angetrieben und durch ein Federspann-und-lösesystem (22) betätigt wird und wobei der Kolben (20) an einem Schaft montiert ist, der axial im zylindrischen Behälter (19) ausgerichtet ist, während der Behälter (19) durch einen Verbindungsdurchgang (23) an der Feinzerstäubungskammer (1) angebracht ist.

14. Inhalator nach Anspruch 1, **dadurch gekennzeichnet, dass** die Feinzerstäubungskammer (1), die Kapselleerungskammer (2) und der Kapselhalter (3) eng aneinander angebracht sind.

15. Inhalator nach Anspruch 1, **dadurch gekennzeichnet, dass** der Querschnitt der Feinzerstäubungskammer (1) auf der Höhe des Aerosolauslasses (7) größer als der Querschnitt der Feinzerstäubungskammer (1) auf der Höhe des Lufteinlasses (6) ist.

## Revendications

1. Inhalateur pour l'administration de médicaments pulvérulents pourvu d'une chambre de pulvérisation dans laquelle est placée la poudre destinée à être inhalée, et la chambre comporte une entrée d'air et une sortie d'air situées sur des parois latérales opposées de la chambre de pulvérisation, **caractérisé en ce que** la chambre de pulvérisation (1) est fixée en sa partie supérieure à une chambre de vidange de capsule (2), qui est couverte par le dessus par un couvercle servant de porte-capsule (3), tandis que l'entrée d'air (6), configurée pour permettre à un flux d'air comprimé d'entrer dans la chambre de pulvérisation (1) de façon tangente par rapport à sa partie inférieure, constitue un passage d'entrée à la source d'air pour la pulvérisation (4), une cloison (8) étant en outre prévue dans la sortie de l'aérosol (7).

2. Inhalateur selon la revendication 1, **caractérisé en ce que** l'entrée d'air (6) consiste en au moins une fente située dans la partie inférieure de la chambre de pulvérisation cylindrique (1) et le long d'une partie de sa circonférence formée à l'intérieur d'un angle d'ouverture (□) qui ne dépasse pas 120°.

3. Inhalateur selon la revendication 1, **caractérisé en ce que** l'entrée d'air (6) consiste en un ensemble d'ouvertures situées dans la partie inférieure de la chambre de pulvérisation cylindrique (1), à l'intérieur d'un secteur qui correspond à un angle d'ouverture (□) non inférieur à 30° et qui ne dépasse pas 120°.

4. Inhalateur selon la revendication 1, **caractérisé en ce que** la partie inférieure de la chambre de pulvérisation (1) est de forme concave.

5. Inhalateur selon la revendication 1, **caractérisé en ce que** l'air comprimé entre dans la chambre de pulvérisation (1) par pulsations depuis la source d'air pour la pulvérisation (4), à travers une vanne d'isolement (5) montée entre l'entrée d'air (6) et la source d'air pour la pulvérisation.

6. Inhalateur selon la revendication 1, **caractérisé en ce que** le volume de l'air introduit dans la chambre de pulvérisation (1), après dilatation dans des conditions normales, dépasse le volume de la chambre de pulvérisation (1).

7. Inhalateur selon la revendication 1, **caractérisé en ce qu'**il y a une cavité (9) dans la surface inférieure du porte-capsule (3), dont la forme correspond à celle de la capsule qui contient le médicament administré.

8. Inhalateur selon la revendication 7, **caractérisé en ce que** la cavité (9) est en position centrale dans le porte-capsule (3).

9. Inhalateur selon la revendication 1, **caractérisé en ce que** la chambre de vidange de capsule (2) est pourvue d'un corps (10), et un outil de cisaillement (11) est monté dans une paroi latérale de celui-ci, tandis que des ouvertures d'entrée d'air extérieur (16) sont formées dans des parois latérales du corps (10) afin d'assurer l'entrée d'un flux d'air extérieur dans l'inhalateur.

10. Inhalateur selon la revendication 9, **caractérisé en ce qu'**un guide (12) pourvu d'un ressort de rappel (14) est logé dans une paroi latérale du corps (10), et l'outil de cisaillement (11) connecté à un bouton (13) placé sur l'extérieur est monté dans le guide (12), et en outre un stabilisateur de la position de la capsule (15) est monté dans la chambre de vidange de capsule (2), et il maintient la capsule en place quand l'outil de cisaillement (11) exerce une pression sur cette dernière.

11. Inhalateur selon la revendication 9, **caractérisé en ce qu'**un guide (12) pourvu d'un ressort de rappel (14) est logé dans une paroi latérale du corps (10), et l'outil de cisaillement (11) connecté à un bouton (13) placé sur l'extérieur est monté dans le guide (12), tandis qu'au-dessus de la chambre de vidange de capsule (2), se trouve un récipient (19) qui libère une bande (18) portant des cloques (17), chaque cloque (17) contenant une dose du médicament administré.

12. Inhalateur selon la revendication 1, **caractérisé en ce que** la source d'air pour la pulvérisation (4) constitue un récipient (19), avec un piston (20) placé dedans et connecté à un ressort moteur (21) et à un système de tension et de libération de ressort (22), tandis que le récipient (19) est relié à la chambre de pulvérisation (1) par l'intermédiaire d'un passage d'entrée (23).

13. Inhalateur selon la revendication 1, **caractérisé en ce que** la source d'air pour la pulvérisation (4) constitue un récipient (19), avec un piston (20) placé dedans, le piston (20) étant entraîné par un ressort moteur (21) et actionné par un système de tension et de libération de ressort (22), et le piston (20) est monté sur un axe aligné axialement dans ledit récipient cylindrique (19), tandis que ledit récipient (19) est relié à la chambre de pulvérisation (1) par l'intermédiaire d'un passage d'entrée (23).

14. Inhalateur selon la revendication 1, **caractérisé en ce que** la chambre de pulvérisation (1), la chambre de vidange de capsule (2) et le porte-capsule (3) sont solidement fixés les uns aux autres.

15. Inhalateur selon la revendication 1, **caractérisé en ce que** la section de la chambre de pulvérisation (1) au niveau de la sortie de l'aérosol (7) est plus grande que la section de la chambre de pulvérisation (1) au niveau de l'entrée d'air (6).
